# EUROPEAN PATENT APPLICATION

(11) **EP 1 652 472 A1**
(43) Date of publication of application: **03.05.2006**
(21) Application number: 04380210.7
(22) Date of filing: 27.10.2004
(51) Int. Cl.: A61B 5/103, A61B 5/107

(54) **Portable manual multiple-measurement device for medical examinations and diagnosis**

(71) Applicant: Luis Mata, Diego, 09004 Burgos (ES)
(72) Inventor: Luis Mata, Diego, 09004 Burgos (ES)
(74) Representative: Manzano Cantos, Gregorio

(57) **Abstract**

A manual and portable multiple-measurement device for medical examination and diagnoses is constituted of an essentially transparent, single laminar body or plate of little thickness, bringing together all the measuring instruments most often used for examination and diagnosis, having reduced dimensions so that it is portable and fits in a pocket. Said transparent single laminar body or plate is provided with graduated rulers on its perimeter, except on the side where the flexible arms or the Velcro holding fastener or the like are hinged; a standard goniometer pivoting on the longer side; a gravitational indicator by way of an inclinometer; flexible hinged arms; the arrangement of a "Perthes" ruler; a magnifying glass facilitating the diagnosis of alterations of the skin, and a scale of circles for measuring the lesions thereof.

## Description

### OBJECT OF THE INVENTION

The manual and portable tool of the invention is indicated and intended in most medical examinations in general and especially in traumatology and orthopedics, in rehabilitation, in podiatry, in examinations, in assessments, i.e. in all those cases in which it is necessary to measure in a simple and quick manner the range, limit or absence of movements, deviations, rotations, displacements or others, especially of the extremities or segments thereof, as well as on radiological images or any other type of images aiding in the diagnosis.

It is therefore a measuring tool which, advantageously, integrates in a single apparatus the instruments which are most often used for traumatological and orthopedic medical examination and diagnosis, in rehabilitation, orthopedics or the like. There are many measuring systems, but none which brings together in a single system the measuring possibilities which the tool of the invention has. In addition, it has a small magnifying glass to better assess skin lesions in daily clinical practice.

With the device of the invention, angles can be measured by means of a goniometer, limb rotations measured with an inclinometer incorporated therein, which is very difficult with a standard goniometer, other linear measurements can be measured in centimeters and inches and carrying out measurements which are difficult to reference is achieved by means of a Perthes ruler.

In order to ensure the simplicity of adaptation to the use and operation conditions, the tool of the invention is simple, light, and of reduced dimensions, whereby becomes a portable tool, being able to be carried in a pocket for the purpose of always having it on hand.

The solution of the invention provides important advantages in comparison with the means used hitherto, although the evolution is aimed towards electronic solutions or computer developments, the integration in the same tool of the different measuring instruments most common in the activity of professional specialists who need to carry out this type of measurements to make an effective diagnosis of certain damages or lesions in the type of diseases previously described.

Advantageously, the object of the invention is easily adapted to the use and operation conditions; it is especially easy-to-use; it has reduced size, because it fits in a pocket; the obtained measurements are reliable.

### BACKGROUND OF THE INVENTION

### DESCRIPTION OF THE INVENTION

The object of the invention is constituted of a laminar body or main plate of little thickness on which measuring elements which measure in centimeters and inches are grouped; it is a transparent body for the purpose of enabling vision of the areas to be measured, and placing it on a certain area of the anatomy of the patient allows aligning the plate with the areas which are to be measured.

Another detail of the invention is that said plate incorporates a graduated ruler preferably located on a side edge of said plate, which ruler rotates around a shaft, by way of a standard goniometer, for measuring angles and which, open at 180°, allows increasing linear measurements along the entire length thereof. To that end, the plate is placed on the proximal portion of the limb or segment with the rotating shaft on the joint to be measured. The rotating ruler or goniometer accompanies the distal segment of the limb to assess its maximum and minimum aperture.

According to the invention, said plate incorporates a gravitational indicator with counterweight as inclinometer with which rotations of the limbs or segments thereof are measured in degrees. It is placed on the limb or segment thereof to be measured starting from a neutral position.

Depending on its placement, it allows assessing the rotations around any shaft.

The plate of the invention may be provided with:
*two flexible hinged arms acting as an extension for the correct coupling of the gravitational indicator and which, when the former is not in use, remain withdrawn on the plate. These arms are placed, encircling the limb or segment thereof in which the rotations are to be measured, allowing the gravitational indicator to remain in the suitable position in order to carry out a correct measuring.
*or a Velcro holding fastener or the like, with which the tool is adapted to the limb or segment thereof in which the rotations are to be measured, allowing the gravitational indicator to remain in the suitable position in order to carry out a correct measuring.

The features of the design of this body allow using the tool like a "Perthes" ruler, integrating it on a squared end of the plate, carrying out measurements which are difficult to reference, such as for example the fibular malleolus in valgus feet. By placing one end of said ruler on the malleolus which is to be projected, this measurement is moved to the point where the ruler is resting. This distance is added and continued, the projection of malleolus being obtained.

Likewise and according to the invention, a magnifying glass facilitating the diagnosis of alterations of the skin is incorporated on the other end of the body of the plate.

In another feature of the invention and in an area aligned with the gravitational indicator, a scale graduated in eccentrically projecting circles is incorporated, allowing measuring the skin lesions which have been located through said magnifying glass or by simple visual deduction.

### BRIEF DESCRIPTION OF THE DRAWINGS

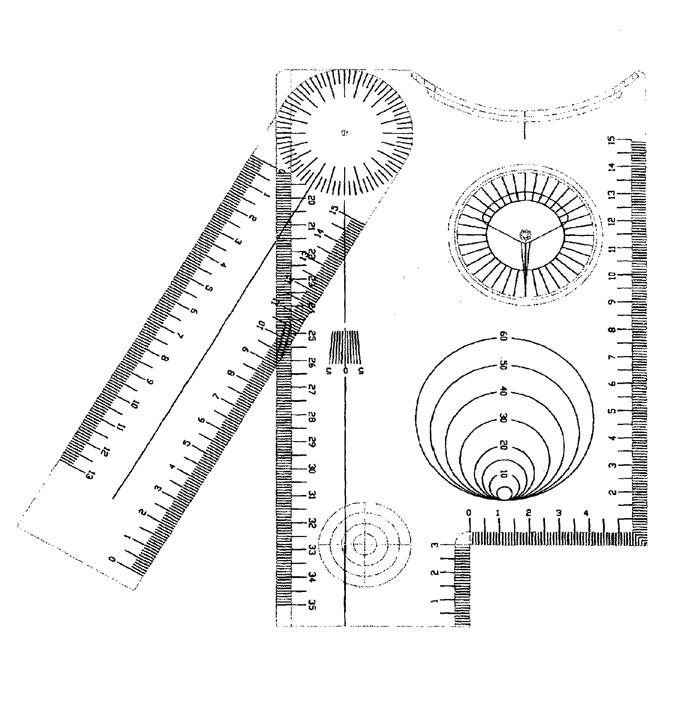

Figure 1 shows an upper plan view with the general development of the laminar body or plate of the invention where, according to the invention, all the localization, measuring and diagnostic instruments are brought together.

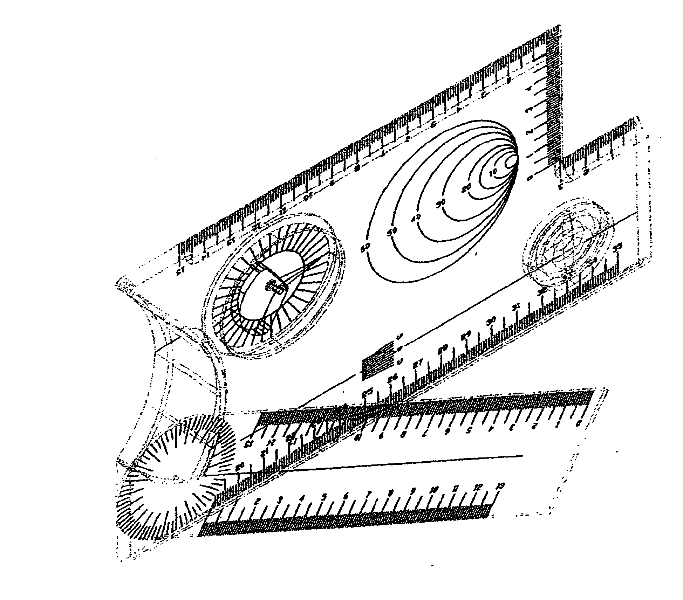

Figure 2 shows an perspective view

### PREFERRED EMBODIMENT OF THE INVENTION

According to said illustrations and in accordance with the references contained therein, a preferred embodiment of the invention would be constituted of...

## Claims

1. A manual and portable multiple-measurement device for medical examination and diagnoses, wherein the instruments measuring in centimeters and inches are brought together, **characterized in that** it is constituted of an essentially transparent single laminar body or plate of little thickness, provided with graduated rulers on its perimeter, except on the side where the flexible arms or the Velcro holding fastener or the like are hinged; a standard goniometer pivoting on the longer side; a gravitational indicator by way of an inclinometer; flexible hinged arms; the arrangement of a "Perthes" ruler; a magnifying glass facilitating the diagnosis of alterations of the skin, and a scale of circles for measuring the lesions thereof.

2. A manual and portable multiple-measurement device for medical examination and diagnoses according to the previous claim, the graduated ruler of the longer side is **characterized in that** it is situated on a longitudinal side, and thereon another one rotating around a shaft in order to measure the angles and which, open at 180°, allows increasing linear measurements, being placed on the proximal portion of the limb or segment with the rotating shaft on the joint to be measured.

3. A manual and portable multiple-measurement device for medical examination and diagnoses according to claim 1, wherein the gravitational indicator is **characterized in that** it is situated in the body of the plate, in the area between where the two flexible arms or the Velcro holding fastener or the like are hinged, and which is provided with a counterweight which allows measuring the rotations of the limbs or segments thereof in degrees, being placed starting from a neutral position.

4. A manual and portable multiple-measurement device for medical examination and diagnoses according to claim 1, the flexible hinged arms are **characterized in that** they are assembled on one end of the plate, located on both sides of the gravitational indicator for the correct coupling of the latter and which remain withdrawn or folded on the plate when the latter is not in use, or instead, a Velcro holding fastener or the like, with which the tool is adapted to the limb or segment thereof in which the rotations are to be measured, allowing the gravitational indicator to remain in the suitable position in order to carry out a correct measuring.

5. A manual and portable multiple-measurement device for medical examination and diagnoses according to claim 4, the "Perthes" ruler is **characterized in that** it is comprised in a squared area on the end opposite to the location of the flexible arms or the Velcro holding fastener or the like, carrying out measurements which are difficult to reference.

6. A manual and portable multiple-measurement device for medical examination and diagnoses according to claim 1, the magnifying glass is **characterized in that** it is specially located in a side position at the same end of the "Perthes" ruler, just at the end of the ruler of the plate corresponding to the goniometer and, when the latter is closed, its movable ruler protects the magnifying glass from scratches.

7. A manual and portable multiple-measurement device for medical examination and diagnoses according to claim 1, the scale of circles is **characterized in that** it is a graduated, asymmetrically developed scale aligned with the gravitational indicator.

## Amended claims

### Amended claims in accordance with Rule 86(2) EPC.

**1.** A manual and portable multiple-measurement device for medical examination and diagnosis, a type of comprising, combination instrument with graduated rulers, a goniometre and an inclinometer, as under FR A 2 094 766 , wherein the instruments measuring in centimetres and millimetres are brought together, principally **characterized** for diagnosis of lesions and malformation of the foot; in that it is constituted of an essentially transparent single laminar body or plate (1) of little thickness, provided with graduated rulers (6) on its perimeter, except on the side where the flexible arms (14, 15) or the Velcro holding fastener or the like are hinged (16); a standard goniometer (2, 3) pivoting on the longer side; a gravitational indicator (9) by way of an inclinometer (11); the arrangement of a "Perthes" ruler (8); a magnifying glass (13) facilitating the diagnosis of alterations of the skin, and a eccentric scale of circles (12) for measuring the lesions thereof.

**2.** A manual and portable multiple-measurement device for medical examination and diagnosis, according to the previous claim 1, the graduated ruler (7) of the longer side is **characterized in that** it is situated on a longitudinal side, and thereon another one rotating around a shaft (6) in order to measure the angles (7) and which, open at 180° (2 + 3), allows increasing linear measurements to be placed on the proximal portion of the limb or segment with the rotating shaft (6) on the joint to be measured.

**3.** A manual and portable multiple-measurement device for medical examination and diagnosis according to claim 1, wherein the gravitational indicator (9) is **characterized in that** it is situated in the body of the plate (1), in the area (16) between where the two flexible arms or the Velcro holding fastens (14, 15) or the like are hinged, and which is provided with a counterweight (10) which allows measuring the rotations of the limbs or segments thereof in degrees (11), when placed from a neutral starting position.

**4.** A manual and portable multiple-measurement device for medical examination and diagnosis according to claim 1, the flexible hinged arms (14, 15) are **characterized in that** they are assembled on one end of the plate (1), located on both sides of the gravitational indicator (9), for the correct coupling of the latter and which remain withdrawn or folded on the plate (1) when the latter is not in use, or instead, a Velcro holding fastener or the like (16), with which the tool is adapted to the limb or segment thereof in which the rotations are to be measured (11), allowing the gravitational indicator (9) to remain in the suitable position in order to carry out a correct measuring.

**5.** A manual and portable multiple-measurement device for medical examination and diagnosis according to claim 4, the "Perthes" ruler (8) is **characterized in that** it is comprised in a squared area on the end opposite to the location of the flexible arms (14,15) or the Velcro holding fastener (16) or the like, carrying out measurements which are difficult to reference.

**6.** A manual and portable multiple-measurement device for medical examination and diagnosis according to claim 1, the magnifying glass (13) is **characterized in that** it is specially located in a side position at the same end of the "Perthes" ruler (8), just at the end of the ruler (8) of the plate (1) corresponding to the goniometer (2, 3) and, when the latter is closed, its movable ruler protects the magnifying glass (13) from scratches.

**7.** A manual and portable multiple-measurement device for medical examination and diagnosis according to claim 1, the scale of circles is **characterized in that** it is a graduated, asymmetrically developed scale (12) that is aligned with the gravitational indicator (10).
